# EUROPEAN PATENT APPLICATION

(11) **EP 4 817 675 A1**
(43) Date of publication of application: **07.10.2026**
(21) Application number: 26169553.0
(22) Date of filing: 31.03.2026
(51) Int. Cl.: A61N 1/39

(54) **MEDICAL DEVICE WITH RECHARGEABLE AND SINGLE-USE POWER SOURCES**

(30) Priority: 31.03.2025 US 202563780679 P; 26.03.2026 US 202619629945
(71) Applicant: STRYKER CORPORATION, Portage, MI 49002-9711 (US)
(72) Inventor: HARVEY, Adam, Portage, 49002-9711 (US)
(74) Representative: Kilburn & Strode LLP

(57) **Abstract**

An example method includes charging, by a first circuit of a medical device, a capacitor by receiving a current from a single-use battery and discharging, by the first circuit of the medical device, an electrical shock to electrodes configured to be disposed on a subject. The example method further includes transmitting, by a second circuit of the medical device drawing a current from a rechargeable battery, a communication signal to an external device; and recharging, by the second circuit of the medical device, the rechargeable battery by receiving a current from a charging source.

## Description

### CROSS-REFERENCE TO RELATED APPLICATION

This application claims priority to U.S. Provisional Application No. 63/780,679, which was filed on March 31, 2025 and is incorporated by reference herein in its entirety.

### BACKGROUND

Various types of medical devices are designed to be used portably. These medical devices utilize portable power sources, such as batteries, to power various components. For instance, a portable external defibrillator is configured to detect physiological parameters of a subject and administer electrotherapy treatments to the subject. A portable chest compression device is configured to administer chest compressions by a motor configured to manipulate a compressor (e.g., a plunger or belt) configured to apply a periodic pressure to the chest of a subject. If the battery of a medical device is depleted, the medical device is unable to monitor or treat subjects.

### BRIEF DESCRIPTION OF THE DRAWINGS

FIG. 1 illustrates an example defibrillator system utilizing different types of power sources.
FIG. 2 is a diagram of an example of a medical device system that utilizes different types of power sources.
FIG. 3 illustrates an example process for operation of a medical device utilizing different types of power sources.
FIG. 4 illustrates an example of an external defibrillator configured to perform various functions described herein.

### DETAILED DESCRIPTION

Various implementations described herein relate to medical devices utilizing both rechargeable and non-rechargeable power sources. In various cases, it may be preferred to utilize a medical device with a disposable accessory that is equipped with a single-use battery. The single-use battery, for instance, can be relatively low-cost and provide a ready power source for sudden, essential functions of the medical device. These essential functions include primary functions, which may relate to monitoring and treating patients. In particular examples, an automated external defibrillator (AED) is configured to be equipped with a disposable and replaceable cartridge that includes both single-use electrode pads and a single-use battery that supports defibrillation functions of the device. The single-use battery may be particularly helpful for supporting defibrillation functions of the AED.

However, during storage of the medical device, it may also be beneficial for the medical device to perform secondary functions that are not directly connected with monitoring or treating a patient. For instance, the medical device may perform automated self-tests, identify its location, or communicate with external devices about various types of information. Utilizing the single-use battery for such additional functions may deplete the single-use battery over time, reducing the likelihood that the medical device will be ready to treat or monitor a patient during a sudden rescue event.

In various implementations of the present disclosure, these and other issues can be addressed by equipping the medical device with a rechargeable battery in addition to the single-use battery. In various cases, the rechargeable battery is configured to supply power to a secondary circuit in the medical device executing secondary functions, whereas the single-use battery is configured to supply power to a primary circuit in the medical device executing primary functions. For instance, the AED may include a rechargeable battery configured to supply power to a non-defibrillation circuit, and may utilize the single-use battery in the disposable cartridge to supply power to a defibrillation circuit. According to various examples, the primary and secondary circuits are electrically isolated from one another. In some cases, a barrier is physically disposed between the primary and secondary circuits. In some examples, components in the primary and secondary circuits can communicate information with one another via inductive, light-based, or other wireless interfaces. Optionally, the single-use battery can provide energy to the rechargeable battery via an inductive interface.

Implementations of the present disclosure will now be described with reference to the accompanying figures.

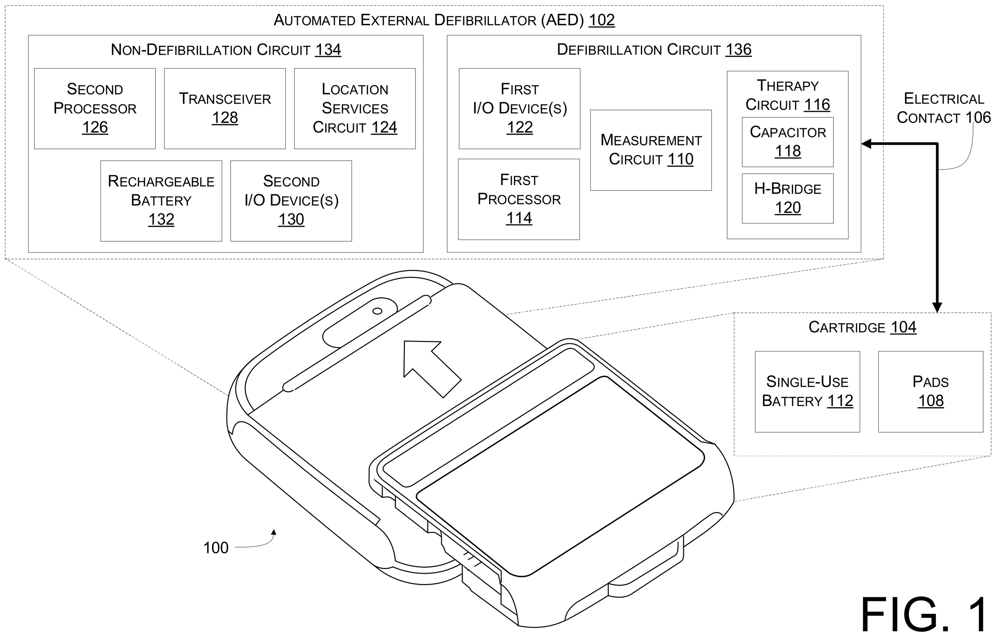

FIG. 1 illustrates an example defibrillator system 100 utilizing different types of power sources. The defibrillator system 100 includes an AED 102 and a cartridge 104. In various implementations the AED 102 is configured to monitor and administer treatments to various subjects, such as patients or other individuals. In various cases, the AED 102 is stored in a location that is accessible by members of the public, including individuals without specific medical training. In various cases, the AED 102 is configured to be stored in a cabinet located in a non-clinical environment, such as in an airport terminal, school, or workplace. In these environments, a subject may suddenly and unexpectedly collapse from a shockable arrhythmia, such as ventricular fibrillation (VF) or pulseless ventricular tachycardia (VT). By accessing and utilizing the AED 102 to monitor and treat the subject, a bystander can prevent the subject from experiencing serious, lasting harm from the shockable arrhythmia.

The AED 102 is configured to be reusable for multiple subjects. However, the AED 102 is configured to be utilized with some single-use (e.g., disposable) components. For example, the cartridge 104 is configured to be removably coupled with the AED 102, and replaced after the AED 102 is utilized with components of the cartridge 104. The cartridge 104 is substantially enclosed in a housing that is configured to be physically coupled with a housing of the AED 102. When the AED 102 and the cartridge 104 are physically coupled together, an electrical contact 106 is formed between the AED 102 and the cartridge 104. In various implementations, some of the components of the AED 102 and the cartridge 104 are configured to be electrically coupled and/or communicatively coupled via the electrical contact 106. In various cases, the cartridge 104 includes pads 108 configured to be disposed on skin of the subject during use.

In an acute rescue scene, a bystander may access the AED 102 coupled with the cartridge 104. The bystander may apply pads 108 in the cartridge 104 to skin of the subject. According to various examples, the pads 108 are configured to be adhered to the skin of the subject, such as skin on the chest of the subject. In various cases, electrodes within the pads 108 are electrically coupled with a measurement circuit 110 in the AED 102. The measurement circuit 110 is powered by a single-use battery 112 within the cartridge 104. The single-use battery 112, for instance, is non-rechargeable. In some examples, the single-use battery 112 includes an alkaline battery, a lithium-metal battery, or a zinc-carbon battery. According to various instances, the single-use battery 112 is preferred over a rechargeable battery for supplying power to at least some components of the AED 102, due to its relatively low cost, ease of use, and limited opportunity for necessary maintenance. It may be relatively simple to maintain readiness of the AED 102 by replacing the cartridge 104 with the single-use battery 112 after use, as opposed to reusing the cartridge 104 multiple times.

In various cases, the measurement circuit 110 is configured to detect an electrocardiogram (ECG) of the subject via the electrodes within the pads 108. For example, the measurement circuit 110 is configured to detect the ECG by drawing power from the single-use battery 112. The measurement circuit 110 is electrically coupled to the pads 108 and the single-use battery 112 via the electrical contact 106 when the cartridge 104 is coupled with the AED 102.

A processor within the AED 102, such as first processor 114, is configured to predict a condition of the subject by analyzing the ECG. For example, the first processor 114 is configured to analyze data indicative of the ECG detected by the measurement circuit 110. In various cases, the first processor 114 determines that the ECG is indicative of VF. In response to identifying the VF, the AED 102 may initiate a treatment protocol.

According to some examples, the treatment protocol utilizes a therapy circuit 116 within the AED 102. In various examples, the therapy circuit 116 includes a capacitor 118. The AED 102, for instance, charges the capacitor 118 by drawing power from the single-use battery 112 in the cartridge 104. When the capacitor 118 is charged, the therapy circuit 116 may discharge the capacitor to electrodes in the pads 108 via an H-bridge 120 within the therapy circuit 116. For instance, the H-bridge 120 causes administration of a multiphasic (e.g., biphasic) electric shock to the pads 108. In some implementations, the VF of the subject can be treated when the multiphasic electric shock is administered to the subject's heart.

To ensure that the AED 102 is usable by an untrained bystander, the AED 102 may include various input/output (I/O) devices, such as at least one first I/O device 122, to implement a user interface. In various examples, the first I/O device(s) 122 include a display (e.g., a touchscreen, a backlit display, one or more light-emitting diodes (LEDs), an LED display, a liquid crystal display (LCD), an organic LED (OLED) display, a quantum dot LED (QLED) display, or other display technologies), one or more speakers, haptic feedback devices (e.g., a device configured to vibrate), or other active elements configured to convey information to the user. The first I/O device(s) 122 may be communicatively coupled with the first processor 114. For instance, the first processor 114 may cause the first I/O device(s) 122 to output instructions to unwrap the pads 108 from packaging, to adhere the pads 108 to the subject (e.g., at specific positions), to administer chest compressions or assisted ventilation to the subject, to avoid contact with the body of the subject (e.g., in advance of administering the electrical shock), to initiate administration of the electrical shock, to check a condition (e.g., a pulse, spontaneous breathing, etc.) of the subject, or the like. In some examples, the first I/O device(s) 122 are configured to detect user input signals from the user. For example, the first I/O device(s) 122 include one or more buttons, touch sensors, dials, keypads, or the like. In some examples, the first I/O device(s) 122 include a power button configured to activate the AED 102, an input device confirming that the user is not touching the subject, a shock button, or the like. In various implementations, the first I/O device(s) 122 are powered by the single-use battery 112, which is connected to the first I/O device(s) 122 via the electrical contact 106.

In various implementations, it may be beneficial for the AED 102 to have additional functions beyond monitoring and treatment functions. For example, it may be helpful for the AED 102 to include a location services circuit 124 configured to detect a location of the AED 102. In various cases, the location services circuit 124 circuit is configured to detect, from multiple external devices, communication signals. The location services circuit 124 may be configured to triangulate the location of the AED 102 by analyzing the received communication signals. For instance, the location services circuit 124 may receive the communication signals from Global Positioning System (GPS) satellites, Global Navigation Seattle System (GLONASS) satellites, BeiDou Navigation Satellite System satellites, Galileo Navigation Satellite System satellites, or a combination thereof. In some cases, the location services circuit 124 transmits communication (e.g., BLUETOOTH^{™}) signals to nearby devices, which report the position of the AED 102 to an external computer (e.g., one or more servers).

According to various cases, the AED 102 may include a processor, such as a second processor 126, that is configured to perform other processing functions of the AED 102. In some examples, the second processor 126 is configured to perform a self-test when the AED 102 is not in use. For example, the second processor 126 may control a circuit (not illustrated) configured to detect a charge level of the single-use battery 112, whether the pads 108 are ready for use (e.g., by determining if an electrical impedance of the electrodes in the pads 108 is within a predetermined range), whether the measurement circuit 110 is ready for use, whether the therapy circuit 116 is ready for use, or whether any other component of the AED 102 may benefit from maintenance or replacement. In various cases, the second processor 126 is configured to generate an alert based on the result of any self-test described herein.

In some examples, it may be valuable for the AED 102 to include a transceiver 128. The transceiver 128, for instance, enables the AED 102 to transmit and/or receive communication signals from external devices. In some cases, the AED 102 may transmit communication signals to report results of a self-test (e.g., based on a charge level of the single-use battery 112 or a state of the pads 108), transmit an alert based on the result of a self-test, report the location of the AED 102, information related to the rescue event (e.g., whether the subject was predicted to have VF, the ECG of the subject, whether one or more electrical shocks have been administered to the subject, or the like), and other information relevant to the state of the AED 102 or the subject. A remote server, for instance, may receive the communication signals and communicate to maintenance professionals whether the cartridge 104 should be replaced or whether the AED 102 is in need of repair. In some examples, the communication signals may be sent to a remote computing device associated with a medical professional, who can initiate coaching, deployment of an emergency response team, or prepare to care for the subject after transport.

In some implementations, the AED 102 also includes at least one I/O device, such as at least one second I/O device 130, configured to serve as an interface with a user regarding functions of the AED 102 that are independent of patient monitoring or treatment. For instance, the second I/O device(s) 130 may include one or more light sources, a display, a speaker, a haptic feedback device, or any combination thereof. In some examples, the second I/O device(s) 130 are configured to output signals indicating a connection status of the transceiver 128, a result of a self-test, an alert associated with a self-test, a location of the AED 102, or any combination thereof. In various cases, the second I/O device(s) 130 include one or more input devices configured to detect, from a user, a request to detect the location of the AED 102 via the location services circuit 124, a detect to transmit a communication signal to an external device via the transceiver 128, to perform a self-test via the second processor 126, or the like.

The AED 102 may have components (e.g., the location services circuit 124, the second processor 126, the transceiver 128, and the second I/O device(s) 130) with various functions that are extraneous to the immediate application of monitoring and care to a subject. However, it may be problematic to power these components with the single-use battery 112. For instance, ongoing location detection and reporting functions of the AED 102 may deplete the single-use battery 112. If the single-use battery 112 is depleted by the time the AED 102 is applied to the subject, the AED 102 may be unable to acutely monitor and treat the subject. Thus, the additional functions of the AED 102 may reduce readiness if they are powered by the same single-use battery 112 utilized to power the monitoring and treatment components of the device.

In various implementations of the present disclosure, the AED 102 includes a rechargeable battery 132 configured to power various secondary components of the AED 102. For instance, the secondary components include any elements of the AED 102 that are not specifically configured to monitor or treat a subject. The rechargeable battery 132 may be part of the AED 102 itself, rather than removably coupled to the AED 102. In some implementations, the AED 102 includes a port configured to connect the rechargeable battery 132 to a charging source, which could be mains current or an external device configured to charge the rechargeable battery 132. In various cases, the rechargeable battery 132 includes a lithium-ion battery, a nickel-metal hydride battery, or any other type of rechargeable battery known in the art.

According to some examples, the rechargeable battery 132 is part of, and provides power to, components within a non-defibrillation circuit 134 of the AED 102. The non-defibrillation circuit 134 includes various elements that are related to functions of the AED 102 that are different than patient monitoring and treatment functions of the AED 102. For example, the non-defibrillation circuit 134 includes the location services circuit 124, the second processor 126, the transceiver 128, the second I/O device(s) 130, and the rechargeable battery. The components within the non-defibrillation circuit 134 are electrically and communicatively connected to one another, for example.

The AED 102 further includes a defibrillation circuit 136 that includes the measurement circuit 110, the first processor 114, the therapy circuit 116, and the first I/O device(s) 122. When the cartridge 104 is physically coupled with the AED 102, the pads 108 and single-use battery 112 are electrically coupled with the defibrillation circuit 136 via the electrical contact 106, such as when the AED 102 is powered on. The single-use battery 112, for instance, supplies power to components within the defibrillation circuit 136.

In various implementations, the non-defibrillation circuit 134 and the defibrillation circuit 136 are distinct from one another. In some cases, the non-defibrillation circuit 134 is electrically and/or physically isolated from the defibrillation circuit 136. For instance, a nonconductive material, such as a housing or other barrier, may be disposed between the non-defibrillation circuit 134 and the defibrillation circuit 136. The housing and/or barrier, in some implementations, includes an electrically insulative material, such as an insulative polymer.

Optionally, the non-defibrillation circuit 134 and the defibrillation circuit 136 are communicatively coupled to one another. For instance, the non-defibrillation circuit 134 may include a component (e.g., a coil) that is inductively coupled with a component (e.g., another coil) within the defibrillation circuit 136. In various cases, one component can induce an electrical signal in the other component via the inductive coupling. In some examples, the non-defibrillation circuit 134 includes a light source that is configured to emit light encoding information that is detected by a sensor in the defibrillation circuit 136, and/or vice versa. Accordingly, the non-defibrillation circuit 134 and the defibrillation circuit 136 may be configured to communicate information, such as self-test results, despite the separation of the non-defibrillation circuit 134 and the defibrillation circuit 136.

In some examples, the single-use battery 112 may be configured to recharge the rechargeable battery 132. For instance, if the results of a self-test indicate that the pads 108 are expired or have already been used, such that the cartridge 104 should be replaced, the defibrillation circuit 136 may be configured to transfer energy from the single-use battery 112 to the rechargeable battery 132 in the non-defibrillation circuit 134. Accordingly, any charge within the single-use battery 112 may be efficiently utilized to power the AED 102 before the cartridge 104 is replaced.

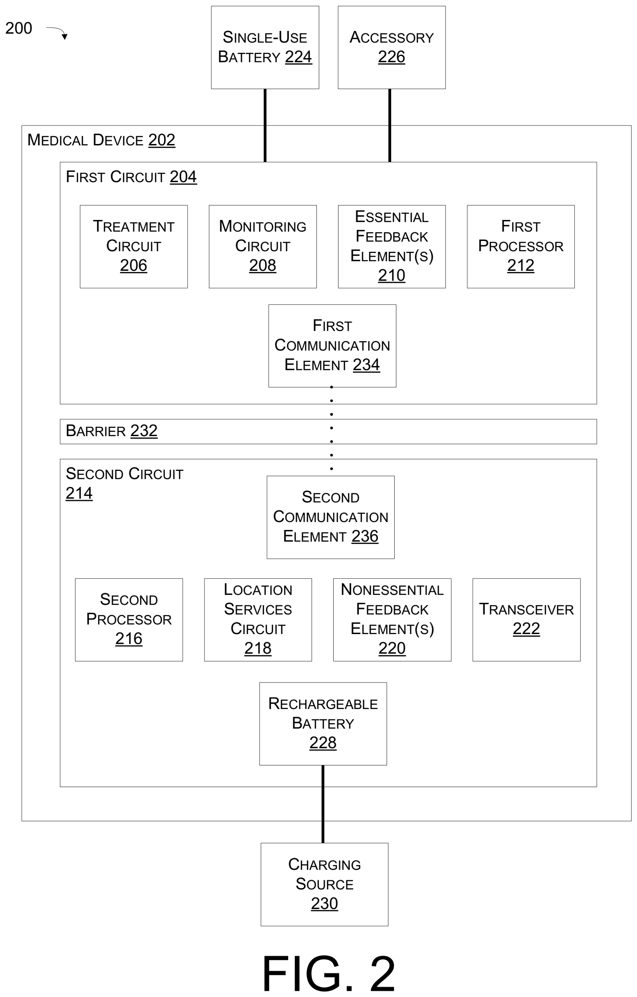

FIG. 2 is a diagram of an example of a medical device system 200 that utilizes different types of power sources. For instance, the medical device system 200 may correspond to the defibrillator system 100 described above with reference to FIG. 1.

The medical device system 200 includes a medical device 202. In various cases, the medical device 202 includes a defibrillator (e.g., an AED or monitor-defibrillator), a mechanical chest compression device, a ventilation device (e.g., a ventilation monitor), a patient monitor, or some other type of medical device configured to monitor and/or administer a treatment to a subject.

The medical device 202 includes a first circuit 204 configured to perform functions related to monitoring and/or treatment of a subject. For example, the first circuit 204 includes a treatment circuit 206 configured to administer a therapy to a subject. For example, the treatment circuit 206 includes an electrotherapy circuit (e.g., a capacitor, an H-bridge, etc.) configured to output one or more electrical shocks and/or pacing pulses to the subject, a motor, an actuator (e.g., configured to move a compressor on the chest of the subject), a pump (e.g., configured to move air in and out of the subject's airway or to administer a medication to the subject), or any combination thereof.

The first circuit 204 optionally includes a monitoring circuit 208 configured to facilitate monitoring of a condition of the subject. In some cases, the monitoring circuit 208 detects one or more parameters of the subject, such as physiological parameters. For example, the monitoring circuit 208 is configured to detect an ECG, a heart rate, a pulse rate, an airway parameter (e.g., a capnograph, a partial pressure of carbon dioxide in the airway, a partial pressure of oxygen in the airway, an airway pressure, a flow rate of air in the airway, etc.), a blood oxygenation (e.g., a pulse oxygenation, a regional oxygenation, a cerebral oxygenation, etc.), a blood pressure (e.g., a systolic blood pressure, a diastolic blood pressure, an instantaneous blood pressure in at least one blood vessel, etc.), a blood flow parameter (e.g., an instantaneous flow rate of blood in one or more blood vessels, etc.), a temperature (e.g., a core temperature), or any combination thereof.

Additionally, in some implementations, the monitoring circuit 208 includes one or more essential feedback elements 210 and a first processor 212. In various implementations, the essential feedback element(s) 210 are configured to output treatment recommendations, prompts, alerts, indications of parameters, indications of conditions, warnings, instructions, or other feedback related to caring for subjects being monitored and/or treated by the medical device 202. The first processor 212 is communicatively coupled with the treatment circuit 206, the monitoring circuit 208, the essential feedback element(s) 210, or a combination thereof. In various examples, the first processor 212 is configured to analyze the parameter(s) of the subject, cause the treatment circuit 206 to administer a treatment to the subject, determine conditions of the subject, generate the warnings, generate the instructions, generate the other feedback, or a combination thereof.

The medical device 202 also includes a second circuit 214 configured to perform additional functions. For example, the second circuit 214 includes a second processor 216 configured to perform additional processing functionality, such as executing self-tests of various components of the medical device 202 or components connected with the medical device 202.

The second circuit 214, in various cases, includes a location services circuit 218. In various cases, the location services circuit 218 is configured to detect and/or transmit signals with other devices and to detect the location of the medical device 202 based on the signals. In some examples, the location services circuit 218 is configured to triangulate the location of the medical device 202 based on detecting wireless signals transmitted from satellites, such as GPS satellites.

In various implementations, the second circuit 214 includes one or more nonessential feedback elements 220. The nonessential feedback element(s) 220, for instance, include output devices configured to report a state of the medical device 202 itself to users. In various cases, the nonessential feedback element(s) 220 indicate results of the self-tests and/or the location of the medical device 202. In some cases, the nonessential feedback element(s) 220 outputs a signal that facilitates a user to locate the medical device 202, such as a blinking light or audible signal.

According to some cases, the second circuit 214 includes a transceiver 222 configured to transmit and/or receive communication signals with devices external to the medical device 202. In some examples, the transceiver 222 is configured to transmit communication signals indicating information generated by the first processor 212 (e.g., an indication of the condition of a subject, an indication that a treatment has been administered to the subject, specific parameters or treatment settings associated with the subject, etc.), information generated by the second processor 216 (e.g., results of a self-test), the location of the medical device 202, or any combination thereof. In some examples, the transceiver 222 is configured to receive communication signals requesting execution of one or more functions of the second circuit 214, such as self-tests, location detection, or activation of the nonessential feedback element(s) 220.

In various implementations, the first circuit 204 and the second circuit 214 are configured to utilize different power sources. For example, the first circuit 204 is configured to be electrically coupled with a single-use battery 224. In various cases, the single-use battery 224 is electrically coupled with the first circuit 204 when the medical device 202 transitions to an active state of responding to the condition of a subject. For example, the single-use battery 224 may be connected when the medical device 202 is turned on or otherwise activated by a rescuer. In various implementations, after the single-use battery 224 is used to power the first circuit 204 during a single rescue event (e.g., monitoring or treating a single subject), the single-use battery 224 is designed to be discarded and replaced.

The single-use battery 224, in some examples, is packaged with an accessory 226. In some examples, the single-use battery 224 and the accessory 226 are stored within a cartridge configured to be removably coupled to a housing of the medical device 202. A package containing the single-use battery 224 and the accessory 226 may be disposable. In various cases, the accessory 226 is configured to be utilized by the first circuit 206 to detect one or more parameters of a subject and/or to administer a treatment to the subject. For example, the accessory 226 may include one or more electrodes, a compressor, a drug delivery device (e.g., a needle, an inhaler, etc.), a bag-valve mask (BVM), an airway adapter, a laryngoscope, a gas sensor, a blood oxygenation sensor, a catheter (e.g., configured to be coupled with a blood pressure sensor), a pressure sensor, an ultrasound transducer, a flow sensor, a light sensor, an accelerometer, a gyroscope, or any combination thereof. According to some cases, the accessory 226 is configured to transmit a signal (e.g., an analog signal and/or digital signal) indicative of one or more parameters of the subject to the first circuit 206. In some cases, the first circuit 206 includes one or more elements configured to activate and/or actuate the accessory 226. In some cases, the package containing the single-use battery 224 includes a peelable container that encloses the accessory 226 during storage. According to some cases, the package is ready-for-use as long as the accessory 226 has not been accessed in the package and/or as long as a charge level of the single-use battery 224 is above a threshold.

In contrast to the first circuit 206, the second circuit 214 is powered by a rechargeable battery 228. In some cases, the rechargeable battery 228 is part of the second circuit 214. In various cases, a charge level of the rechargeable battery 228 can be increased when the rechargeable battery 228 is coupled to a charging source 230. The charging source 230 may be a separate device from the medical device 202. In some examples, the charging source 230 includes a mains current power source. For instance, the medical device 202 may include a cord, plug, connector, port, or combination thereof, configured to electrically connect the first circuit 206 to the charging source 230, enabling recharging of the rechargeable battery 228.

To prevent the second circuit 214 from depleting the single-use battery 224, the first circuit 206 and the second circuit 214 are isolated from one another. For instance, there an absence of any conductive path that connects the first circuit 206 to the second circuit 214 within the medical device 202. In various cases, a barrier 232 is configured to physically isolate the first circuit 206 and the second circuit 214. In some examples, the barrier 232 includes at least a portion of a housing enclosing the first circuit 206 and/or at least a portion of the housing enclosing the second circuit 214. In some examples, the barrier 232 includes an electrically insulative and/or dielectric material. For instance, the barrier 232 includes a polymer material.

In some implementations, the first circuit 206 and the second circuit 214 are configured to exchange data. For instance, the first circuit 206 includes a first communication element 234 and the second circuit 214 includes a second communication element 236. In various cases, the first communication element 234 and the second communication element 236 are configured to exchange data wirelessly.

According to some example, the first communication element 234 includes a first coil and the second communication element 236 includes a second coil. The first coil and the second coil may be inductively coupled, in some implementations. For example, a current in the first coil changes changing respect to time induces a change in a magnetic field associated with the first coil also with respect to time. In various cases, the changing magnetic field causes a voltage to be induced in the second coil. Accordingly, the first coil may be configured to induce an electrical signal via a magnetic coupling between the first coil and the second coil, or vice versa. In various cases, data can be encoded in the electrical signal induced in the second coil by the first coil.

In some cases, the first communication element 234 and the second communication element 236 include at least one light source and at least one light detector, such that the first communication element 234 and the second communication element 236 are configured to perform light-based communication. In various examples, at least a portion of the barrier 232 includes an optically transmissive material. For instance, an example light source may be configured to transmit a light signal through the barrier 232 to an example light detector. A color (frequency), phase, timing, pulse pattern, intensity, or a combination thereof of the light signal may encode data.

According to some instances, the first communication element 234 and the second communication element 236 include at least one transmitter configured to transmit wireless signals and at least one receiver configured to receive the wireless signals. For instance, the first communication element 234 and the second communication element 236 include one or more antennas, one or more transducers, or the like. The wireless signals may include electromagnetic signals (e.g., near-field communication (NFC) signals), pressure waves (e.g., ultrasound signals), or any combination thereof. In various examples, a timing, a frequency, a phase, or a combination thereof of the wireless signals may encode data.

Various types of information can be exchanged between the first communication element 234 and the second communication element 236. For example, the first communication element 234 may be configured to report a state of the single-use battery 224, the accessory 226, or a package containing the single-use battery 224 and the accessory 226. In some implementations, the first communication element 234 transmits, to the second communication element 236, a communication signal indicating that the single-use battery 224 and/or the accessory 226 are ready to be replaced. For instance, in response to a rescue event in which the first circuit 206 has been activated, the first communication element 234 may transmit, to the second communication element 236, a communication signal indicating that the single-use battery 224, the accessory 226, or a package containing the single-use battery 224 and the accessory 226 is ready for replacement. In some examples, the communication signal indicates a charge level of the single-use battery 224 and/or whether the package has been opened. According to some cases, the first communication element 234 is configured to transmit a communication signal indicating parameter(s) detected from a subject and/or one or more treatments administered to the subject. For instance, the communication signal may indicate whether the subject exhibited signs of VF, an amount of time that the subject remained in VF, a number of electric shocks administered to the subject, or the like. In some implementations, the second communication element 236 transmits a communication signal to the first communication element 234 that causes the first circuit 204 to activate or charge a capacitor in the treatment circuit 206 (e.g., if the transceiver 222 receives a communication signal from an external device indicating that the medical device 202 is about to be utilized to monitor and/or treat a subject).

Optionally, the first communication element 234 and the second communication element 236 are configured to transfer power from the single-use battery 224 to the rechargeable battery 228 under specific conditions. For instance, after the single-use battery 224 is utilized to monitor and/or treat a subject, the single-use battery 224 may be ready for replacement. However, it may be wasteful to discard the single-use battery 224 if the single-use battery 224 has a charge level over a threshold. In various examples, in response to monitoring and/or treating a subject, the first circuit 206 is configured to draw current from the single-use battery 224 in order to transfer the energy from the single-use battery 24 to the second circuit 214. For instance, the energy can be transferred via an inductive coupling between the first communication element 234 and the second communication element 236. The second circuit 214, for instance, can utilize the energy for various uses, such as to supply power to the second processor 216, the location services circuit 218, the nonessential feedback element(s) 220, the transceiver 222, or a combination thereof. In some aspects, the energy from the single-use battery 224 is used to recharge the rechargeable battery 228. Accordingly, residual energy in the single-use battery 224 can be efficiently utilized before the single-use battery 224 is discarded.

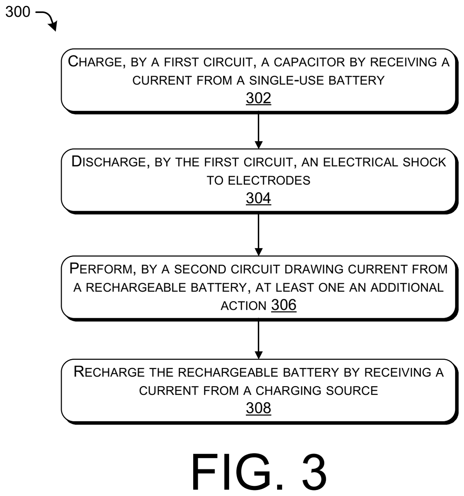

FIG. 3 illustrates an example process 300 for operation of a medical device utilizing different types of power sources. The process 300 is performed by an entity, such as the medical device (e.g., the medical device 202, a mechanical chest compression device, a ventilation device, or the like), a defibrillator (e.g., the AED 102), a medical device system (e.g., the system 100 and/or the system 200), at least one computing device, at least one processor, or any combination thereof.

At 302, the entity charges, by a first circuit, a capacitor by receiving a current from a single-use battery. In various cases, the single-use battery includes an alkaline battery, a lithium-metal battery, or a zinc-carbon battery.

At 304, the entity discharges, by the first circuit, an electrical shock to electrodes. In various cases, the electrical shock is discharged by discharging the capacitor. In some cases, the electrodes and the single-use battery are part of a disposable package, such as a cartridge configured to be physically coupled with the medical device. For instance, when the disposable package is physically connected to the medical device,

At 306, the entity performs, by a second circuit drawing current from a rechargeable battery, at least one additional action. In various examples, the rechargeable battery is physically and/or electrically isolated from the first circuit. In some cases, a barrier is disposed between the first circuit and the second circuit. For instance, the rechargeable battery is part of the second circuit, which is physically and/or electrically isolated from the first circuit, which may include the capacitor. The rechargeable battery, for instance, includes a lithium-ion battery or a nickel-metal hydride battery.

Various types of additional actions are performed by the entity in 306. In some examples, the entity transmits, by a transceiver in the second circuit powered by the rechargeable battery, a communication signal to an external device. In some examples, the communication signal indicates a status of the single-use battery or the status of the electrodes. For example, in examples in which the first circuit is communicatively coupled with the second circuit, the first circuit may indicate, to the second circuit, that the single-use battery and/or the electrodes have been used. For instance, the first circuit may output, by an optical transmitter (e.g., a light source) an optical signal indicating a status of the single-use battery and/or the electrodes, wherein the second circuit detects the light signal. In some examples, the first circuit induces, by a coil of the first circuit, an electrical signal in a coil of the second circuit, wherein the electrical signal encodes the status of the single-use battery and/or the electrodes. For instance, if the coils are inductively coupled, generating a time-varying current in one coil produces an electrical signal in the other coil. In some cases, the first circuit indicates the status of the single-use battery and/or the electrodes in response to concluding a rescue event (e.g., when the medical device is powered off). According to some examples, the transceiver transmits a communication signal indicating one or more parameters of a subject monitored and/or treated by the medical device. In some cases, the transceiver transmits a communication signal indicating a location of the medical device.

In some implementations, the entity outputs, by an output device in the second circuit powered by the rechargeable battery, an instruction to a user. For instance, the instruction is to replace the single-use battery or the electrodes. According to some cases, the instruction is to connect a single-use battery and/or electrodes to the medical device, such as to connect a cartridge containing an unused single-use battery and/or electrodes to the medical device. In some cases, the instruction is to repair or provide a maintenance check of the medical device.

According to some cases, the entity determines, by a location services circuit in the second circuit, communication signals transmitted from external devices. For instance, the external devices include satellites. In various implementations, the second circuit is configured to determine the location of the entity based on the communication signals. In some examples, the location services circuit transmits communication signals to one or more other external devices, which may report the location of the medical device based on the transmitted communication signals.

At 308, the entity recharges the rechargeable battery by receiving a current from a charging source. For instance, the second circuit is connected with a charging source, such as an external device or an electrical grid, that provides a current used to recharge the rechargeable battery.

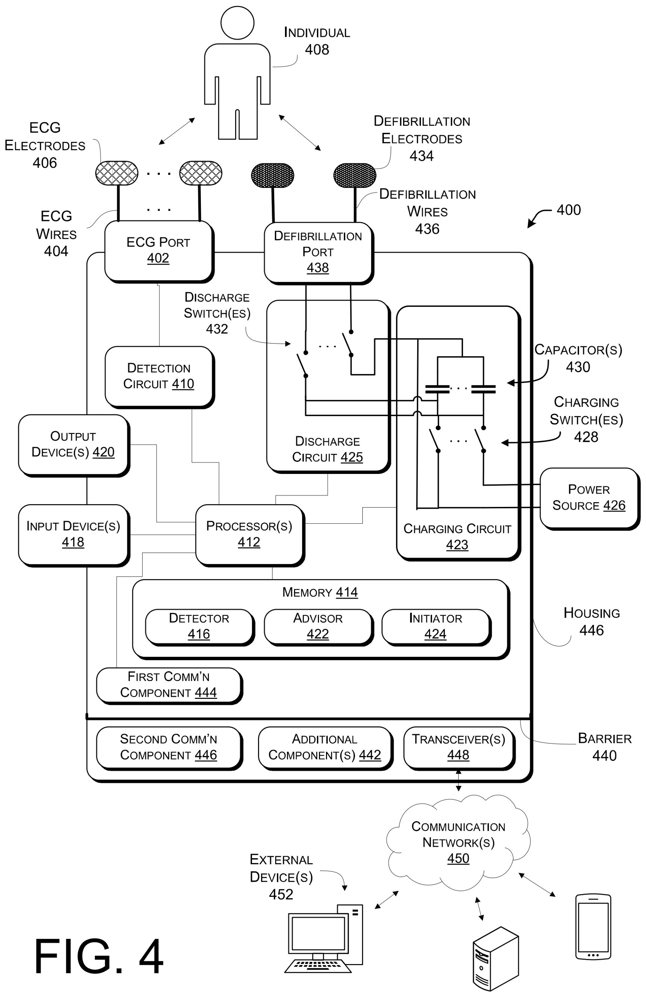

FIG. 4 illustrates an example of an external defibrillator 400 configured to perform various functions described herein. For example, the external defibrillator 400 is the AED 102 described above with reference to FIG. 1 and/or the medical device 202 described above with reference to FIG. 2.

The external defibrillator 400 includes an ECG port 402 connected to multiple ECG wires 404. In some cases, the ECG wires 404 are removeable from the ECG port 402. For instance, the ECG wires 404 are plugged into the ECG port 402 via connectors. The ECG wires 404 are connected to ECG electrodes 406, respectively. In various implementations, the ECG electrodes 406 are disposed on different locations on an individual 408. A detection circuit 410 is configured to detect relative voltages between the ECG electrodes 406. These voltages are indicative of the electrical activity of the heart of the individual 408.

In various implementations, the ECG electrodes 406 are in contact with the different locations on the skin of the individual 408. In some examples, a first one of the ECG electrodes 406 is placed on the skin between the heart and right arm of the individual 408, a second one of the ECG electrodes 406 is placed on the skin between the heart and left arm of the individual 408, and a third one of the ECG electrodes 406 is placed on the skin between the heart and a leg (either the left leg or the right leg) of the individual 408. In these examples, the detection circuit 410 is configured to measure the relative voltages between the first, second, and third ECG electrodes 406. Respective pairings of the ECG electrodes 406 are referred to as "leads," and the voltages between the pairs of ECG electrodes 406 are known as "lead voltages." In some examples, more than three ECG electrodes 406 are included, such that 5-lead or 12-lead ECG signals are detected by the detection circuit 410.

The detection circuit 410 includes at least one analog circuit, at least one digital circuit, or a combination thereof. The detection circuit 410 receives the analog electrical signals from the ECG electrodes 406, via the ECG port 402 and the ECG wires 404. In some cases, the detection circuit 410 includes one or more analog filters configured to filter noise and/or artifact from the electrical signals. The detection circuit 410 includes an analog-to-digital (ADC) in various examples. The detection circuit 410 generates a digital signal indicative of the analog electrical signals from the ECG electrodes 406. This digital signal can be referred to as an "ECG signal" or an "ECG."

In some cases, the detection circuit 410 further detects an electrical impedance between at least one pair of the ECG electrodes 406. For example, the detection circuit 410 includes, or otherwise controls, a power source that applies a known voltage (or current) across a pair of the ECG electrodes 406 and detects a resultant current (or voltage) between the pair of the ECG electrodes 406. The impedance is generated based on the applied signal (voltage or current) and the resultant signal (current or voltage). In various cases, the impedance corresponds to respiration of the individual 408, chest compressions performed on the individual 408, and other physiological states of the individual 408. In various examples, the detection circuit 410 includes one or more analog filters configured to filter noise and/or artifact from the resultant signal. The detection circuit 410 generates a digital signal indicative of the impedance using an ADC. This digital signal can be referred to as an "impedance signal" or an "impedance."

The detection circuit 410 provides the ECG signal and/or the impedance signal one or more processors 412 in the external defibrillator 400. In some implementations, the processor(s) 412 includes a central processing unit (CPU), a graphics processing unit (GPU), both CPU and GPU, or other processing unit or component known in the art.

The processor(s) 412 is operably connected to memory 414. In various implementations, the memory 414 is volatile (such as random access memory (RAM)), nonvolatile (such as read only memory (ROM), flash memory, etc.) or some combination of the two. The memory 414 stores instructions that, when executed by the processor(s) 412, causes the processor(s) 412 to perform various operations. In various examples, the memory 414 stores methods, threads, processes, applications, objects, modules, any other sort of executable instruction, or a combination thereof. In some cases, the memory 414 stores files, databases, or a combination thereof. In some examples, the memory 414 includes, but is not limited to, RAM, ROM, electrically erasable programmable read-only memory (EEPROM), flash memory, or any other memory technology. In some examples, the memory 414 includes one or more of CD-ROMs, digital versatile discs (DVDs), content-addressable memory (CAM), or other optical storage, magnetic cassettes, magnetic tape, magnetic disk storage or other magnetic storage devices, or any other medium which can be used to store the desired information and which can be accessed by the processor(s) 412 and/or the external defibrillator 400. In some cases, the memory 414 at least temporarily stores the ECG signal and/or the impedance signal.

In various examples, the memory 414 includes a detector 416, which causes the processor(s) 412 to determine, based on the ECG signal and/or the impedance signal, whether the individual 408 is exhibiting a particular heart rhythm. For instance, the processor(s) 412 determines whether the individual 408 is experiencing a shockable rhythm that is treatable by defibrillation. Examples of shockable rhythms include VF and ventricular tachycardia (V-Tach). In some examples, the processor(s) 412 determines whether any of a variety of different rhythms (e.g., asystole, sinus rhythm, atrial fibrillation (AF), etc.) are present in the ECG signal.

The processor(s) 412 is operably connected to one or more input devices 418 and one or more output devices 420. Collectively, the input device(s) 418 and the output device(s) 420 function as an interface between a user and the defibrillator 400. The input device(s) 418 is configured to receive an input from a user and includes at least one of a keypad, a cursor control, a touch-sensitive display, a voice input device (e.g., a microphone), a haptic feedback device (e.g., a gyroscope), or any combination thereof. The output device(s) 420 includes at least one of a display, a speaker, a haptic output device, a printer, or any combination thereof. In various examples, the processor(s) 412 causes a display among the input device(s) 418 to visually output a waveform of the ECG signal and/or the impedance signal. In some implementations, the input device(s) 418 includes one or more touch sensors, the output device(s) 420 includes a display screen, and the touch sensor(s) are integrated with the display screen. Thus, in some cases, the external defibrillator 400 includes a touchscreen configured to receive user input signal(s) and visually output physiological parameters, such as the ECG signal and/or the impedance signal.

In various implementations, the input device(s) 418 further include, or are otherwise connected to, one or more physiological sensors. The physiological sensor(s), for instance, are configured to detect one or more physiological parameters of the individual 408. Examples of the physiological sensor(s) include a blood pressure sensor (e.g., a blood pressure cuff, invasive blood pressure sensor, or the like), an airway sensor (e.g., a sensor configured to detect a partial pressure of CO₂ and/or O₂ in an airway of the individual 408), a blood oxygenation sensor (e.g., a pulse oximeter, regional oxygenation sensor, or the like), a thermometer, a pulse sensor, a blood flow sensor (e.g., an ultrasound transducer configured to detect blood flow using Doppler-based techniques), an airway pressure sensor, or any combination thereof. The input device(s) 418, in some cases, includes one or more sensors configured to detect other characteristics of the individual 408. For example, the input device(s) 418 includes an accelerometer, gyroscope, microphone, or any combination thereof. In various implementations, the processor(s) 412 is configured to assess a condition of the individual 408 by analyzing data derived from signals detected by the input device(s) 418.

In some examples, the memory 414 includes an advisor 422, which, when executed by the processor(s) 412, causes the processor(s) 412 to generate advice and/or control the output device(s) 420 to output the advice to a user (e.g., a rescuer). In some examples, the processor(s) 412 provides, or causes the output device(s) 420 to provide, an instruction to perform CPR on the individual 408. In some cases, the processor(s) 412 evaluates, based on the ECG signal, the impedance signal, or other physiological parameters, CPR being performed on the individual 408 and causes the output device(s) 420 to provide feedback about the CPR in the instruction. According to some examples, the processor(s) 412, upon identifying that a shockable rhythm is present in the ECG signal, causes the output device(s) 420 to output an instruction and/or recommendation to administer a defibrillation shock to the individual 408.

The memory 414 also includes an initiator 424 which, when executed by the processor(s) 412, causes the processor(s) 412 to control other elements of the external defibrillator 400 in order to administer a defibrillation shock to the individual 408. In some examples, the processor(s) 412 executing the initiator 424 selectively causes the administration of the defibrillation shock based on determining that the individual 408 is exhibiting the shockable rhythm and/or based on an input from a user (received, e.g., by the input device(s) 418. In some cases, the processor(s) 412 causes the defibrillation shock to be output at a particular time, which is determined by the processor(s) 412 based on the ECG signal and/or the impedance signal.

The processor(s) 412 is operably connected to a charging circuit 423 and a discharge circuit 425. In various implementations, the charging circuit 423 is connected to a power source 426 and includes one or more charging switches 428 and one or more capacitors 430. The power source 426 includes, for instance, a battery. In some cases, the power source 426 includes a single-use, nonrechargeable battery. In some examples, the power source 426 is stored in a package (e.g., a cartridge) with the ECG electrodes 406.

The processor(s) 412 initiates a defibrillation shock by causing the power source 426 to charge at least one capacitor among the capacitor(s) 430. For example, the processor(s) 412 activates at least one of the charging switch(es) 428 in the charging circuit 423 to complete a first circuit connecting the power source 426 and the capacitor to be charged. Then, the processor(s) 412 causes the discharge circuit 425 to discharge energy stored in the charged capacitor across a pair of defibrillation electrodes 434, which are in contact with the individual 408. For example, the processor(s) 412 deactivates the charging switch(es) 428 completing the first circuit between the capacitor(s) 430 and the power source 426, and activates one or more discharge switches 432 completing a second circuit connecting the charged capacitor 430 and at least a portion of the individual 408 disposed between defibrillation electrodes 434.

The energy is discharged from the defibrillation electrodes 434 in the form of a defibrillation shock. For example, the defibrillation electrodes 434 are connected to the skin of the individual 408 and located at positions on different sides of the heart of the individual 408, such that the defibrillation shock is applied across the heart of the individual 408. The defibrillation shock, in various examples, depolarizes a significant number of heart cells in a short amount of time. The defibrillation shock, for example, interrupts the propagation of the shockable rhythm (e.g., VF or VT) through the heart. In some examples, the defibrillation shock is 200 J or greater with a duration of about 0.015 seconds. In some cases, the defibrillation shock has a multiphasic (e.g., biphasic) waveform. The discharge switch(es) 432 are controlled by the processor(s) 412, for example. In various implementations, the defibrillation electrodes 434 are connected to defibrillation leads 436. The defibrillation wires 436 are connected to a defibrillation port 438, in implementations. According to various examples, the defibrillation wires 436 are removable from the defibrillation port 438. For example, the defibrillation wires 436 are plugged into the defibrillation port 438. In some cases, the defibrillation electrodes 434 are stored in the same package as the power source 426.

In various implementations, the defibrillator 400 includes multiple isolated circuits. For example, a barrier 440 is disposed between the circuits of the defibrillator 400. In some cases, the barrier 440 physically and/or electrically isolates the circuits of the defibrillator 400. For instance, the barrier 440 prevents a conductive path from directly extending between the circuits of the defibrillator 400.

One of the isolated circuits, including various aforementioned components of the defibrillator 400, performs functions related to monitoring and treatment of the individual 408. In contrast, another isolated circuit performs non-monitoring and non-treatment functions of the defibrillator 400. For example, this circuit includes one or more additional components 442 configured to perform various functions related to self-tests, location detection, outputting instructions (e.g., maintenance instructions) before or after the individual 408 is connected to the defibrillator 400, and the like. In various implementations, the additional component(s) 442 include a power source that is different from the power source 426. For example, the additional component(s) 442 include a rechargeable battery. In some examples, the additional component(s) 442 include an additional processor that is separate from the processor(s) 412.

In some implementations, the processor(s) 412 is communicatively coupled with a first communication component 444. The first communication component 444, for instance, is configured to communicate data or other information to a second communication component 446. In some implementations, the first communication component 444 and the second communication component 446 are configured to communicate the data and/or other information via an inductive coupling, an optical coupling, or some other wireless interface between the first communication component 444 and the second communication component 446. Accordingly, various data available to the processor(s) 412 and the additional component(s) 442 can be exchanged across the barrier 440.

In various implementations, the circuit includes one or more transceivers 448 that transmit and/or receive data over one or more communication networks 450. For example, the transceiver(s) 448 includes a network interface card (NIC), a network adapter, a local area network (LAN) adapter, or a physical, virtual, or logical address to connect to the various external devices and/or systems. In various examples, the transceiver(s) 448 includes any sort of wireless transceivers capable of engaging in wireless communication (e.g., radio frequency (RF) communication). For example, the communication network(s) 450 includes one or more wireless networks that include a 3^{rd} Generation Partnership Project (3GPP) network, such as a Long Term Evolution (LTE) radio access network (RAN) (e.g., over one or more LTE bands), a New Radio (NR) RAN (e.g., over one or more NR bands), or a combination thereof. In some cases, the transceiver(s) 448 includes other wireless modems, such as a modem for engaging in WI-FI^{®}, WIGIG^{®}, WIMAX^{®}, BLUETOOTH^{®}, or infrared communication over the communication network(s) 450.

The defibrillator 400 is configured to transmit and/or receive data (e.g., ECG data, impedance data, data indicative of one or more detected heart rhythms of the individual 408, data indicative of one or more defibrillation shocks administered to the individual 408, results of self-tests, indications that one or more accessories are ready for replacement, etc.) with one or more external devices 452 via the communication network(s) 450. The external devices 452 include, for instance, mobile devices (e.g., mobile phones, smart watches, etc.), Internet of Things (IoT) devices, medical devices, computers (e.g., laptop devices, servers, etc.), or any other type of computing device configured to communicate over the communication network(s) 450. In some examples, the external device(s) 452 is located remotely from the defibrillator 400, such as at a remote clinical environment (e.g., a hospital). According to various implementations, the processor(s) 412 causes the transceiver(s) 448 to transmit data to the external device(s) 452. In some cases, the transceiver(s) 448 receives data from the external device(s) 452 and the transceiver(s) 448 provide the received data to the processor(s) 412 for further analysis.

In various implementations, the external defibrillator 400 also includes a housing 454 that at least partially encloses other elements of the external defibrillator 400. For example, the housing 454 encloses the detection circuit 410, the processor(s) 412, the memory 414, the charging circuit 423, the transceiver(s) 448, or any combination thereof. In some cases, the input device(s) 418 and output device(s) 420 extend from an interior space at least partially surrounded by the housing 454 through a wall of the housing 454. In various examples, the housing 454 acts as a barrier to moisture, electrical interference, and/or dust, thereby protecting various components in the external defibrillator 400 from damage.

In some implementations, the external defibrillator 400 is an AED operated by an untrained user (e.g., a bystander, layperson, etc.) and can be operated in an automatic mode. In automatic mode, the processor(s) 412 automatically identifies a rhythm in the ECG signal, makes a decision whether to administer a defibrillation shock, charges the capacitor(s) 430, discharges the capacitor(s) 430, or any combination thereof. In some cases, the processor(s) 412 controls the output device(s) 420 to output (e.g., display) a simplified user interface to the untrained user. For example, the processor(s) 412 refrains from causing the output device(s) 420 to display a waveform of the ECG signal and/or the impedance signal to the untrained user, in order to simplify operation of the external defibrillator 400.

In some examples, the external defibrillator 400 is a monitor-defibrillator utilized by a trained user (e.g., a clinician, an emergency responder, etc.) and can be operated in a manual mode or the automatic mode. When the external defibrillator 400 operates in manual mode, the processor(s) 412 cause the output device(s) 420 to display a variety of information that may be relevant to the trained user, such as waveforms indicating the ECG data and/or impedance data, notifications about detected heart rhythms, and the like.

### EXAMPLE CLAUSES

The following clauses provide various implementations of the present disclosure. However, the scope of the disclosure is not limited to any of the clauses listed below.
1. An automated external defibrillator (AED), including: a cartridge including: a single-use battery; and electrodes configured to be disposed on skin of a subject; a first circuit including: a discharge circuit including: an H-bridge configured to be electrically connected to the electrodes; and a capacitor configured to: charge by receiving a charging current from the single-use battery; and in response to charging, discharge an electrical shock to the electrodes via the H-bridge; a port configured to receive, from an external source, a recharging current from the external source; a second circuit including: a rechargeable battery electrically connected to the port and configured to charge by receiving the recharging current; and a transceiver powered by the rechargeable battery, the transceiver being configured to: transmit, to an external device, a communication signal indicating a status of the cartridge; and a housing enclosing the first circuit and the second circuit, the housing being configured to removably couple with the cartridge.
2. The AED of clause 1, wherein the second circuit further includes a processor powered by the rechargeable battery and configured to: determine the status of the cartridge by determining a charge level of the single-use battery.
3. The AED of clause 1 or 2, wherein the second circuit further includes a display powered by the rechargeable battery and configured to: visually present an indication of the status of the cartridge.
4. A medical device, including: a first circuit including: a discharge circuit including: a capacitor configured to: charge by receiving a current from a single-use battery; and in response to charging, discharge an electrical shock to electrodes configured to be disposed on a subject; and a second circuit including: a rechargeable battery; and an additional element powered by the rechargeable battery.
5. The medical device of clause 4, further including: a housing configured to be removably coupled with a cartridge including the single-use battery or the electrodes.
6. The medical device of clause 4 or 5, wherein the single-use battery includes an alkaline battery, a lithium-metal battery, or a zinc-carbon battery.
7. The medical device of any of clauses 4 to 6, wherein the discharge circuit further includes an H-bridge electrically connected between the capacitor and the electrodes.
8. The medical device of any of clauses 4 to 7, wherein the rechargeable battery includes a lithium-ion battery or a nickel-metal hydride battery.
9. The medical device of any of clauses 4 to 8, wherein the additional element includes: a display configured to visually present an instruction to a user.
10. The medical device of any of clauses 4 to 9, wherein the additional element includes: a processor configured to determine a status of the single-use battery or a status of the electrodes.
11. The medical device of clause 10, wherein the additional element further includes: a transceiver configured to transmit a communication signal indicating the status of the single-use battery or the status of the electrodes.
12. The medical device of any of clauses 4 to 11, wherein the additional element includes: a location services circuit configured to: receive communication signals from external devices; and determine a location of the medical device by analyzing the communication signals.
13. A method, including: charging, by a first circuit of a medical device, a capacitor by receiving a current from a single-use battery; in response to charging the capacitor, discharging, by the first circuit of the medical device, an electrical shock to electrodes configured to be disposed on a subject; transmitting, by a second circuit of the medical device drawing a current from a rechargeable battery, a communication signal to an external device; and recharging, by the second circuit of the medical device, the rechargeable battery by receiving a current from a charging source.
14. The method of clause 13, wherein the capacitor is electrically isolated from the rechargeable battery.
15. The method of clause 13 or 14, wherein the single-use battery includes an alkaline battery, a lithium-metal battery, or a zinc-carbon battery.
16. The method of any of clauses 13 to 15, wherein the rechargeable battery includes a lithium-ion battery or a nickel-metal hydride battery.
17. The method of any of clauses 13 to 16, wherein the communication signal further indicates a status of the single-use battery or a status of the electrodes.
18. The method of any of clauses 13 to 17, wherein the charging source includes the single-use battery.
19. The method of any of clauses 13 to 18, wherein the first circuit is inductively coupled with the second circuit, the method further including: determining that a charge level of the single-use battery is below a threshold; in response to determining that the charge level of the single-use battery is below the threshold: generating, in the first circuit by the rechargeable battery in the second circuit, an induced current by generating a time-varying current in the first circuit; and recharging, by the first circuit, the capacitor using the induced current.
20. The method of any of clauses 13 to 19, further including: visually present, by the second circuit of the medical device drawing the current from the rechargeable battery, an instruction to a user.
21. The method of clause 20, wherein the instruction includes an instruction to connect the electrodes or the single-use battery to the medical device.
22. The method of any of clauses 13 to 21, the communication signal being a first communication signal, the method further including: detecting, by the second circuit of the medical device drawing the current from the rechargeable battery, second communication signals transmitted by satellites; determining, by the second circuit of the medical device, a location of the medical device, wherein the first communication signal further indicates the location of the medical device.
23. An automated external defibrillator (AED), including: electrodes configured to be disposed on a subject; a defibrillation circuit including: a discharge circuit including: an H-bridge electrically connected to the electrodes; and a capacitor configured to: charge by receiving a current from a power source; and in response to charging, discharge an electrical shock to the electrodes via the H-bridge; and a first coil; and a non-defibrillation circuit electrically isolated from the defibrillation circuit, the non-defibrillation circuit including: a second coil inductively coupled with the first coil, the first coil being configured to induce, in the second coil, an electrical signal indicating a status of the electrodes; and a transceiver configured to transmit, to an external device, a communication signal indicating the status of the electrodes.
24. The AED of clause 23, wherein the defibrillation circuit further includes: a detection circuit configured to detect, from the electrodes, an electrical signal indicating an electrocardiogram (ECG) of the subject; and a processor configured to: determine that the ECG is indicative of ventricular fibrillation (VF) or ventricular tachycardia (VT); and in response to determining that the ECG is indicative of VF or VT, cause the discharge circuit to charge the capacitor or to discharge the electrical shock.
25. The AED of clause 23 or 24, wherein the non-defibrillation circuit further includes: a light source configured to output a light signal indicating the status of the electrodes.
26. A medical device, including: a first circuit including: a discharge circuit including: a capacitor configured to: charge by receiving a current from a power source; and in response to charging, discharge an electrical shock to electrodes configured to be disposed on a subject; and a second circuit electrically isolated from the first circuit and including an output device or a processor.
27. The medical device of clause 26, wherein the first circuit includes the output device, the output device including: a display or a speaker configured to output an instruction to a user; or a light source indicating a status of the electrodes.
28. The medical device of clause 26 or 27, wherein the second circuit includes the output device, the output device including a transceiver configured to transmit a communication signal indicating a status of the medical device, a status of the power source, a status of the electrodes, or a status of the subject.
29. The medical device of any of clauses 26 to 28, wherein the processor is configured to: cause the discharge circuit to charge the capacitor; and cause the discharge circuit to discharge the capacitor.
30. The medical device of any of clauses 26 to 29, further including: a barrier configured to electrically isolate the first circuit from the second circuit.
31. The medical device of any of clauses 26 to 30, wherein the first circuit further includes: an optical transmitter powered by the power source and configured to output a light signal indicative of a status of the power source or a status of the electrodes, wherein the second circuit further includes: an optical receiver configured to: detect the light signal; and output, to the processor, an electrical signal indicating the light signal, and wherein the processor is configured to: determine the status of the power source or the status of the electrodes by analyzing the electrical signal; and cause the output device to output an indication of the status of the power source or the status of the electrodes.
32. The medical device of any of clauses 26 to 31, wherein the first circuit further includes a first coil, wherein the second circuit further includes a second coil inductively coupled to the first coil, the first coil being configured to induce, in the second coil, an electrical signal indicating a status of the power source or a status of the electrodes, and wherein the processor is configured to:
   determine the status of the power source or the status of the electrodes by analyzing the electrical signal; and cause the output device to output an indication of the status of the power source or the status of the electrodes.
33. The medical device of any of clauses 26 to 32, further including: a housing enclosing the first circuit and the second circuit and being configured to be removably coupled with a cartridge including the power source or the electrodes.
34. The medical device of any of clauses 26 to 33, wherein the power source includes a single-use battery, and wherein the second circuit includes a rechargeable battery configured to power the output device or the processor.
35. A method, including: charging, by a capacitor of a first circuit of a medical device, by receiving a current from a power source; in response to charging the capacitor, discharging, by the first circuit of the medical device, an electrical shock to electrodes configured to be disposed on a subject; determining, by a second circuit of the medical device that is electrically isolated from the first circuit of the medical device, a status of the electrodes; and transmitting, by the second circuit of the medical device, a communication signal indicating the status of the electrodes.
36. The method of clause 35, further including: outputting, by the second circuit of the medical device, an indication of the status of the electrodes.
37. The method of clause 36, wherein outputting the indication of the status of the electrodes includes: transmitting, by the second circuit of the medical device to an external device, a communication signal including the indication of the status of the electrodes.
38. The method of any of clauses 35 to 37, further including: outputting, by an optical transmitter of the first circuit of the medical device, a light signal indicative of the status of the electrodes, wherein determining, by the second circuit of the medical device, the status of the electrodes includes detecting the light signal.
39. The method of any of clauses 35 to 38, further including: inducing, by a first coil of the first circuit of the medical device, an electrical signal in a second coil of the second circuit of the medical device, wherein determining, by the second circuit of the medical device, the status of the electrodes includes analyzing the electrical signal.
40. The method of any of clauses 35 to 39, further including: receiving, by a housing of the medical device, a cartridge including the power source or the electrodes.
41. The method of any of clauses 35 to 40, wherein the power source includes a single-use battery.
42. The method of any of clauses 35 to 41, further including: detecting, by the second circuit of the medical device, a location of the medical device,
   wherein the communication signal further indicates the location of the medical device.

### CONCLUSION

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be used for realizing implementations of the disclosure in diverse forms thereof.

As will be understood by one of ordinary skill in the art, each implementation disclosed herein can comprise, consist essentially of or consist of its particular stated element, step, or component. Thus, the terms "include" or "including" should be interpreted to recite: "comprise, consist of, or consist essentially of." The transition term "comprise" or "comprises" means has, but is not limited to, and allows for the inclusion of unspecified elements, steps, ingredients, or components, even in major amounts. The transitional phrase "consisting of" excludes any element, step, ingredient or component not specified. The transition phrase "consisting essentially of" limits the scope of the implementation to the specified elements, steps, ingredients or components and to those that do not materially affect the implementation. As used herein, the term "based on" is equivalent to "based at least partly on," unless otherwise specified.

Unless otherwise indicated, all numbers expressing quantities, properties, conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques. When further clarity is required, the term "about" has the meaning reasonably ascribed to it by a person skilled in the art when used in conjunction with a stated numerical value or range, i.e. denoting somewhat more or somewhat less than the stated value or range, to within a range of ±20% of the stated value; ±19% of the stated value; ±18% of the stated value; ±17% of the stated value; ±16% of the stated value; ±15% of the stated value; ±14% of the stated value; ±13% of the stated value; ±12% of the stated value; ±11% of the stated value; ±10% of the stated value; ±9% of the stated value; ±8% of the stated value; ±7% of the stated value; ±6% of the stated value; ±5% of the stated value; ±4% of the stated value; ±3% of the stated value; ±2% of the stated value; or ±1% of the stated value.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing implementations (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate implementations of the disclosure and does not pose a limitation on the scope of the disclosure. No language in the specification should be construed as indicating any non-claimed element essential to the practice of implementations of the disclosure.

Groupings of alternative elements or implementations disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain implementations are described herein, including the best mode known to the inventors for carrying out implementations of the disclosure. Of course, variations on these described implementations will become apparent to those of ordinary skill in the art upon reading the foregoing description. The inventors expect skilled artisans to employ such variations as appropriate, and the inventors intend for implementations to be practiced otherwise than specifically described herein. Accordingly, the scope of this disclosure includes all modifications and equivalents of the subject matter recited in the claims appended hereto as permitted by applicable law. Moreover, any combination of the above-described elements in all possible variations thereof is encompassed by implementations of the disclosure unless otherwise indicated herein or otherwise clearly contradicted by context.

## Claims

1. A medical device, comprising:
a first circuit comprising:
a discharge circuit comprising:
a capacitor configured to:
charge by receiving a current from a single-use battery; and
in response to charging, discharge an electrical shock to electrodes configured to be disposed on a subject; and
a second circuit comprising:
a rechargeable battery; and
an additional element powered by the rechargeable battery.

2. The medical device of claim 1, further comprising:
a housing configured to be removably coupled with a cartridge comprising the single-use battery or the electrodes.

3. The medical device of claim 1 or 2, wherein the single-use battery comprises an alkaline battery, a lithium-metal battery, or a zinc-carbon battery, and
wherein the rechargeable battery comprises a lithium-ion battery or a nickel-metal hydride battery.

4. The medical device of any of claims 1 to 3, wherein the discharge circuit further comprises an H-bridge electrically connected between the capacitor and the electrodes.

5. The medical device of any of claims 1 to 4, wherein the additional element comprises:
a display configured to visually present an instruction to a user.

6. The medical device of any of claims 1 to 5, wherein the additional element comprises:
a processor configured to determine a status of the single-use battery or a status of the electrodes.

7. The medical device of claim 6, wherein the additional element further comprises:
a transceiver configured to transmit a communication signal indicating the status of the single-use battery or the status of the electrodes.

8. The medical device of any of claims 1 to 7, wherein the additional element comprises:
a location services circuit configured to:
receive communication signals from external devices; and
determine a location of the medical device by analyzing the communication signals.

9. A method, comprising:
charging, by a first circuit of a medical device, a capacitor by receiving a current from a single-use battery;
in response to charging the capacitor, discharging, by the first circuit of the medical device, an electrical shock to electrodes configured to be disposed on a subject;
transmitting, by a second circuit of the medical device drawing a current from a rechargeable battery, a communication signal to an external device; and
recharging, by the second circuit of the medical device, the rechargeable battery by receiving a current from a charging source.

10. The method of claim 9, wherein the capacitor is electrically isolated from the rechargeable battery.

11. The method of claim 9 or 10, wherein the single-use battery comprises an alkaline battery, a lithium-metal battery, or a zinc-carbon battery, and
wherein the rechargeable battery comprises a lithium-ion battery or a nickel-metal hydride battery.

12. The method of any of claims 9 to 11, wherein the communication signal further indicates a status of the single-use battery or a status of the electrodes.

13. The method of any of claims 9 to 12, wherein the first circuit is inductively coupled with the second circuit, the method further comprising:
determining that a charge level of the single-use battery is below a threshold;
in response to determining that the charge level of the single-use battery is below the threshold:
generating, in the first circuit by the rechargeable battery in the second circuit, an induced current; and
recharging, by the first circuit, the capacitor using the induced current.

14. The method of any of claims 9 to 13, further comprising:
visually present, by the second circuit of the medical device drawing the current from the rechargeable battery, an instruction to a user,
wherein the instruction comprises an instruction to connect the electrodes or the single-use battery to the medical device.

15. The method of any of claims 9 to 14, the communication signal being a first communication signal, the method further comprising:
detecting, by the second circuit of the medical device drawing the current from the rechargeable battery, second communication signals transmitted by satellites;
determining, by the second circuit of the medical device, a location of the medical device,
wherein the first communication signal further indicates the location of the medical device.
